(19) Europäisches Patentamt
European Patent Office
Office européen des brevets

(11) Veröffentlichungsnummer: **0 077 439**
**B1**

(12) # EUROPÄISCHE PATENTSCHRIFT

(45) Veröffentlichungstag der Patentschrift: **24.09.86**

(51) Int. Cl.[4]: **C 07 C 175/00,** C 07 F 9/54

(21) Anmeldenummer: **82106661.0**

(22) Anmeldetag: **23.07.82**

(54) **Verfahren zur Herstellung von Cyclohexenderivaten, sowie ein neues Ausgangsprodukt und neue Zwischenprodukte in diesem Verfahren.**

(30) Priorität: **16.10.81 CH 6626/81**

(43) Veröffentlichungstag der Anmeldung: **27.04.83 Patentblatt 83/17**

(45) Bekanntmachung des Hinweises auf die Patenterteilung: **24.09.86 Patentblatt 86/39**

(84) Benannte Vertragsstaaten: **AT BE CH DE FR GB IT LI NL**

(56) Entgegenhaltungen:
**EP-A-0 031 875**
**DE-A-2 653 838**

**HELVETICA CHIMICA ACTA, Band 65, Fasc. 3, Nr. 89, 1982, Seiten 958-967, Schweizerische Chemische Gesellschaft, Basel, CH., E. WIDMER et al.: "Technische Verfahren zur Synthese von Carotinoiden und verwandten Verbindungen aus 6-Oxo-isophoron. VII. Synthese von Rhodoxanthin und (3RS,3'RS)-Zeaxanthin aus der C15-Ringkomponente"**

(73) Patentinhaber: **F. HOFFMANN- LA ROCHE & CO. Aktiengesellschaft, CH- 4002 Basel (CH)**

(72) Erfinder: **Widmer, Erich, Dr., Mittelweg 47, CH- 4142 Münchenstein (CH)**
Erfinder: **Zell, Reinhard, Dr., Im Zwären, CH- 4118 Rodersdorf (CH)**

(74) Vertreter: **Cottong, Norbert A., Grenzacherstrasse 124 Postfach 3255, CH- 4002 Basel (CH)**

**EP 0 077 439 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft ein neues Verfahren zur Herstellung von Cyclohexenderivaten, nämlich von Cyclohexenderivaten, welche als Zwischenprodukte für die Herstellung von Rhodoxanthin bzw. Zeaxanthin geeignet sind, sowie ein Verfahren zur Herstellung von Rhodoxanthin bzw. von Zeaxanthin selbst. Die Erfindung betrifft ferner ein neues Ausgangsprodukt und neue Zwischenprodukte in diesem Verfahren.

Unter dem Ausdruck Zeaxanthin ist im Rahmen der vorliegenden Erfindung das (3RS, 3'RS)-Zeaxanthin zu verstehen.

Rhodoxanthin ist ein natürlich vorkommendes Carotinoid, welches u.a. als Lebensmittelfarbstoff verwendet werden kann. Auch Zeaxanthin, dessen (3R, 3'R)-Antipode in der Natur vorkommt, kann als Lebensmittelfarbstoff dienen, z.B. zur Eidotterpigmentierung.

Bisher bekannte Synthesen zur Herstellung dieser beiden Carotinoide benötigen etwa 10 bis 18 Stufen. Mittels des erfindungsgemässen Verfahrens können nunmehr diese Substanzen auf wesentlich einfachere Art hergestellt werden.

Das erfindungsgemässe Verfahren ist nun dadurch gekennzeichnet, dass man die Verbindung der Formel

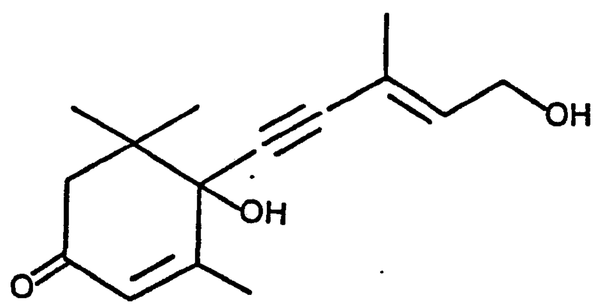

I

in einem stark basischen, homogenen wässrig-organischen Lösungsmittelgemisch mit Zink in die Verbindung der Formel

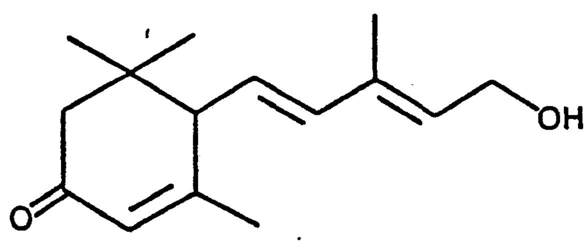

II

überführt, dass man, gewünschtenfalls, die Verbindung der Formel II, nach Ueberführung in das Phosphoniumsalz der Formel

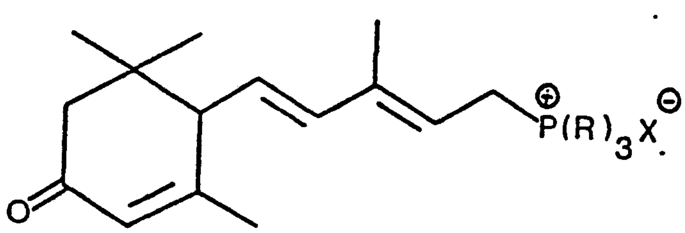

III

worin R Phenyl und X Chlor, Brom oder jod bedeuten, mit dem Dialdehyd der Formel

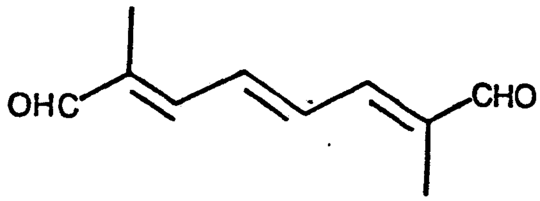

IV

zur Verbindung der Formel

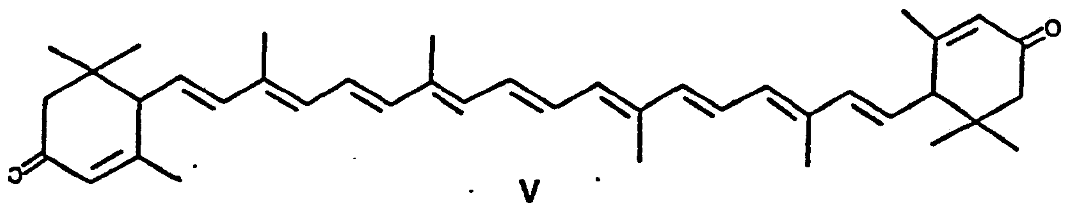

V

umsetzt, dass man, gewünschtenfalls, die Verbindung der Formel V zu Rhodoxanthin der Formel

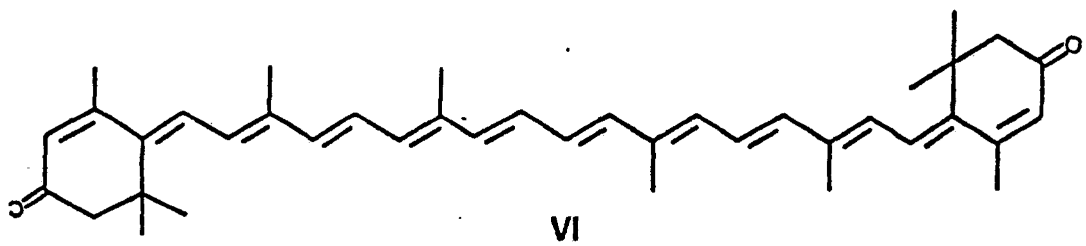

VI

dehydriert und dass man, gewünschtenfalls, dieses Rhodoxanthin zu Zeaxanthin der Formel

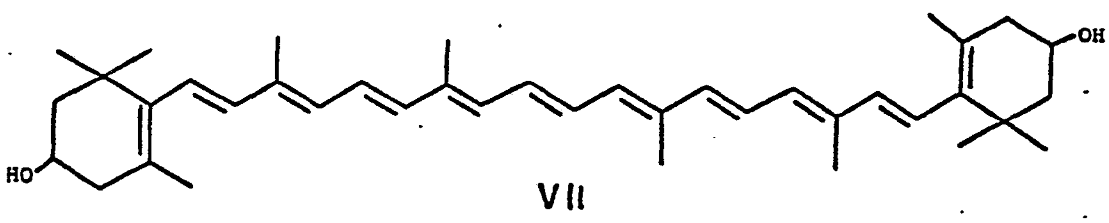

VII

reduziert.

Die Verbindungen der Formeln II und III sind neu und ebenfalls Gegenstand der vorliegenden Erfindung.

Die Verbindung der Formel I ist bekannt aus DE-A-2 653 838.

Die Ueberführung der Verbindung der Formel I in diejenige der Formel II stellt eine reduktive Eliminierung der angulären Hydroxygruppe und gleichzeitige Partialreduktion der Dreifachbindung dar.

Diese Ueberführung erfolgt erfindungsgemäss mit Zink in einem stark basischen, homogenen, wässrig-organischen Lösungsmittelgemisch. Diese Reaktion kann sowohl mit Zink allein, als auch mit Zink in Gegenwart eines Komplexbildners durchgeführt werden. Als Komplexbildner kommen im Rahmen der vorliegenden Erfindung insbesondere Alkalicyanide, wie Lithium-, Natrium- oder Kaliumcyanid, in Frage. Weiterhin kommen Aethylendiamintetraessigsäure (EDTA), Aethylendiamin, Nitrilotriessigsäure, Oxalsäure, o-Phenanthrolin und dergleichen in Frage. Bevorzugt sind hierbei stark komplexierende Substanzen, d.h. solche mit pK-Werten für die Zinkkomplexe in wässriger Lösung grösser als 9.

Die Umsetzung erfolgt wie erwähnt unter stark basischen Bedingungen, insbesondere bei pH-Werten von etwa 13 bis 14, vorzugsweise von etwa 13,5-14 und besonders bevorzugt bei pH 14. Diese pH-Werte können in an sich bekannter Weise erzielt werden, beispielsweise durch Zugabe einer anorganischen Base, wie etwa Natrium- oder Kaliumhydroxid und dergleichen. Bei Durchführung der Reaktion in Gegenwart eines Komplexbildners können diese Bedingungen unter Umständen auch bereits durch diesen Komplexbildner erzielt werden.

Die in dem Verfahren verwendete Menge Zink beträgt zweckmässig von etwa 2 bis etwa 10 Mol und vorzugsweise von etwa 5 bis etwa 8 Mol pro Mol der Verbindung der Formel I. Sofern ein Komplexbildner zugesetzt wird, erfolgt dies vorzugsweise in einer Menge von etwa 0,1 bis etwa 1 Mol und insbesondere in einer Menge von etwa 0,5 bis etwa 0,8 Mol pro Mol der Verbindung der Formel I.

Für das wässrig-organische Lösungsmittelgemisch kommen, im Rahmen der vorliegenden Erfindung, inerte, mit Wasser mischbare organische Lösungsmittel in Frage, insbesondere niedere Alkanole mit I-4 Kohlenstoffatomen, nämlich Methanol, Acthanol, n-Propano1, iso-Propanol, n-Butanol. Weiterhin können hier Dioxan, Tetrahydrofuran, Aceton, Dimethylsulfoxid, Dimethylformamid und dergleichen genannt werden. Bevorzugt sind jedoch die niederen Alkanole, insbesondere Methanol, Aethanol und n-Propanol. Das Verhältnis Lösungsmittel zu Wasser ist begrenzt durch die Löslichkeit der Verbindung der Formel I im speziellen Lösungsmittel-Wassergemisch. Zweckmässig liegt das Verhältnis jedoch zwischen etwa 4:1 bis 1:4 (v/v).

Die Umsetzung kann mit oder ohne Schutzgasatmosphäre durchgeführt werden. Vorzugsweise arbeitet man unter einer Inertgasatmosphäre, wie etwa Stickstoff, Argon und dergleichen. Weiterhin erfolgt die Umsetzung zweckmässig bei einer Temperatur von etwa 0° C bis etwa 50° C, vorzugsweise bei etwa 15° C bis etwa 30° C und insbesondere bei etwa Raumtemperatur.

Bei der Ueberführung der Verbindung der Formel II in das Phosphoniumsalz der Formel III wird in der Verbindung der Formel II zunächst die allylische Hydroxylgruppe gegen Halogen ausgetauscht. Dieser Austausch kann in an sich bekannter Weise mittels Halogenwasserstoff (HCl, HBr oder HJ) in wässriger Lösung (z.B 37%ig, 48%ig oder 57%ig) erfolgen. Der Austausch kann bei Temperaturen zwischen etwa -20° C und etwa +25° C erfolgen, vorzugsweise bei etwa 0° C. Als Lösungsmittel für die Durchführung der Reaktion kann ein für derartige Austauschreaktionen geeignetes Lösungsmittel verwendet werden, beispielsweise ein chlorierter Kohlenwasserstoff, wie Methylenchlorid oder Chloroform und dergleichen.

Die Umsetzung des so erhaltenen Halogenids zum Phosphoniumsalz der Formel III kann in an sich bekannter Weise erfolgen. Zweckmässig erfolgt die Umsetzung mit einem Triarylphosphin, insbesondere mit Triphenylphosphin, in einem geeigneten inerten organischen Lösungsmittel, wie etwa einem chlorierten Kohlenwasserstoff, z.B. Methylenchlorid oder Chloroform oder einem Ester niederer Carbonsäuren mit 1 bis 4 Kohlenstoffatomen, wie Aethylformiat, Aethylacetat usw. Weiterhin erfolgt die Umsetztung vorzugsweise in einer Inertgasatmosphäre und bei etwa Raumtemperatur oder auch bei erhöhter Temperatur. Die Temperatur ist jedoch keine kritische Grösse in dieser Reaktion.

Die Umsetzung des phosphoniumsalzes der Formel III mit dem Dialdehyd der Formel IV zur Verbindung der Formel V kann in an sich bekannter Weise, d.h. unter den bei Wittig-Reaktionen üblichen Bedingungen durchgeführt werden. Zweckmassig erfolgt die Umsetzung in einem chlorierten Kohlenwasserstoff, wie z.B. Methylenchlorid oder Chloroform und in Gegenwart einer Base, wie z.B. Natriummethylat. Es ist hierbei vorteilhaft, die Base nicht auf einmal, sondern langsam und kontinuierlich zu dem Reaktionsgemisch zu geben.

Die Dehydrierung der Verbindung der Formel V zum Rhodoxanthin der Formel VI kann in an sich bekannter Weise durchgeführt werden, beispielsweise gemäss der von Kuhn und Brockmann in Ber. <u>66</u>, 1319 (1933) beschriebenen Oxidation von Dihydrorhodoxanthin zu Rhodoxanthin.

Die Reduktion von Rhodoxanthin der Formel VI zu Zeaxanthin der Formel VII kann ebenfalls in an sich bekannter Weise durchgeführt werden, beispielsweise gemäss Karrer und Solmssen, Helv.Chim.Acta <u>18</u>, 477 (1935).

Die in dem erfindungsgemässen Verfahren als Ausgangsmaterial verwendete Verbindung der Formel I ist neu und ebenfalls Gegenstand der vorliegenden Erfindung. Sie kann ausgehend vom Ketoisophoron der Formel

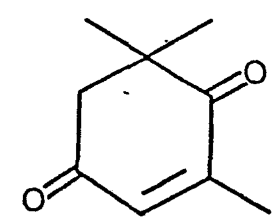

VIII

durch Umsetzung mit der Verbindung der Formel

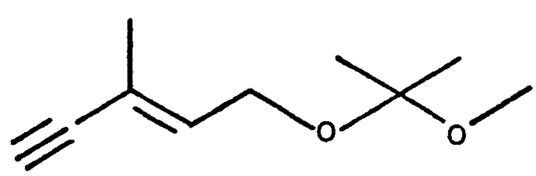

IX

hergestellt werden.

Diese Umsetzung erfolgt zweckmässig über das Lithium- oder Magnesiumsalz der Verbindung IX in einem für Organometallverbindungen inerten organischen Lösungsmittel, wie z.B. offene oder cyclische Aether, wie Diäthyläther, Dioxan, Tetrahydrofuran oder auch aromatische Kohlenwasserstoffe, wie Benzol, Toluol und dergleichen, oder auch in flüssigem Ammoniak. Die Umsetzung erfolgt vorzugsweise bei einer Temperatur von etwa -50° C bis etwa Raumtemperatur. Das bei der Umsetzung eingesetzte Ketoisophoron muss in Stellung 3 geschützt sein, z.B. in Form des Lithiumenolates oder als Monoketal und dergleichen.

**Beispiel 1**

In einem 1,5-Liter-Sulfierkolben, versehen mit Rührer, Thermometer und Begasungsaufsatz, wurden unter Argon 44,3 g (0,178 Mol) 4-Hydroxy-4-(5-hydroxy-3-methyl-3-penten-1-ynyl)-3,5,5-trimethyl-2-cyclohexen-l-on in 800 ml n-Propanol/Wasser (1:1 v/v) gelöst, mit 5,88 g Natriumcyanid (0,12 Mol) und 77,4 g Zinkpulver (1,18 g-Atome) versetzt und unter gelegentlichem leichtem Kühlen bis zum vollständigen Umsatz bei Raumtemperatur kräftig gerührt (ca. 5 Stunden). Die Suspension wurde dann unter Argon abgenutscht und der Rückstand dreimal mit je 100 ml Methylenchlorid und zweimal mit je 100 ml entionisiertem Wasser gewaschen. Das Filtrat wurde mit Hilfe von 50 ml Methylenchlorid in einen 1-Liter-Scheidetrichter $S_1$ gegeben. Zwei weitere 1-Liter-Scheidetrichter ($S_2$ und $S_3$) wurden mit je 100 ml gesättigter Kochsalzlösung beschickt. Hierauf wurden der Reihe nach die organische Phase aus $S_1$ sowie zwei Portionen zu je 100 ml Methylenchlorid unter gutem Durchmischen durch die drei Scheidetrichter $S_1$-$S_3$ geschickt. Die organischen Phasen wurden vereint und über 50 g Natriumsulfat getrocknet. Hierauf wurde abgenutscht, der Rückstand zweimal mit je 50 ml Methylenchlorid gewaschen und die Filtrate am Rotationsverdampfer am Wasserstrahlvakuum bei einer Badtemperatur von 40°C bis zur Gewichtskonstanz getrocknet. Man erhielt 47,9 g Rohprodukt, welches zwecks Reinigung an Kieselgel adsorbiert und mit Hexan/Aether chromatographiert wurde. Man erhielt so 33,9 g (81,3%) 4-(5-Hydroxy-3-methyl-1,3-pentadienyl)-3,5,5-trimethyl-2-cyclohexen-1-on in Form eines Oeles. Die Struktur dieser Verbindung wurde durch Mikroanalyse, NMR-, IR- und MS-Spektren eindeutig charakterisiert.

Das als Ausgangsmaterial verwendete 4-Hydroxy-4-(5-hydroxy-3-methyl-3-penten-1-ynyl)-3,5,5-trimethyl-2-cyclohexen-1-on kann wie folgt hergestellt werden:

In einem 750-ml-Sulfierkolben, versehen mit Rührer, Thermometer, Ammoniakkühler und einem 100-ml-Tropftrichter mit Druckausgleich und Begasungsaufsatz, wurden 250 ml Ammoniak kondensiert und dann mit 0,1 g Eisen(III)nitrat versetzt. Hierauf wurden in ca. 15 Minuten insgesamt 3,2 g Lithiumdraht (0,46 g-Atome) in Portionen zugesetzt. Das Gemisch wurde bei -40°C bis zum vollständigen Umsatz zu $LiNH_2$ weitergerührt (ca. 20 Minuten). Anschliessend wurde unter Kühlen mit einem Aceton/$CO_2$-Bad bei -40°C eine Lösung von 77,4 g Aceton-methyl-3-methyl-2-penten-4-ynyl-acetal in 20 ml absolutem Aether in ca. 20 Minuten zugetropft, gefolgt von 30,4 g 2,6,6-Trimethyl-2-cyclohexen-1,4-dion in 20 ml absolutem Aether. Das Gemisch wurde bei -40°C bis zum vollständigen Umsatz weitergerührt (ca. 2,5 Stunden). Nun wurden 200 ml absoluter Aether zugesetzt und anschliessend Ammoniak mit Hilfe eines Warmwasser-Bades ausgetrieben. Sobald das Gemisch Raumtemperatur erreicht hatte, wurde mit einem Eisbad wieder auf 0°C abgekühlt und durch tropfenweisen Zusatz von 100 ml entionisiertem Wasser hydrolysiert. Zur weiteren Aufarbeitung wurde das Reaktionsgemisch mit Hilfe von 100 ml Aether in einen 1-Liter-Scheidetrichter $S_1$ gespült. Zwei weitere 500-ml-Scheidetrichter ($S_2$ und $S_3$) wurden mit je 200 ml Aether beschickt. Hierauf wurden der Reihe nach und unter gutem Durchmischen zunächst die wässrige Phase aus $S_1$ sowie drei Portionen zu je 100 ml halbgesättigter Kochsalzlösung durch die drei Scheidetrichter $S_1$ bis $S_3$ geschickt. Die organischen Extrakte wurden vereint und über 50 g Natriumsulfat getrocknet. Hierauf wurde abgenutscht, das Trocknungsmittel auf der Nutsche mit 50 ml Aether nachgewaschen und die Filtrate im Rotationsverdampfer am Wasserstrahlvakuum bei 30°C eingeengt. Der Rückstand wurde anschliessend im Rotationsverdampfer mit Trockeneiskühler am Feinvakuum (ca. 0,1 mbar) bei 65°C bis zur Gewichtskonstanz eingeengt. Man erhielt so 62 g (96,8%) des Umsetzungsproduktes in Form eines dunklen Oeles.

Diese 62 g Umsetzungsprodukt wurden zur Hydrolyse 5 in 100 ml Aceton aufgenommen und bei 0°C unter Rühren und Begasen mit Argon tropfenweise in 15 Minuten mit 50 ml 3N Schwefelsäure versetzt. Das schwarze Reaktionsgemisch wurde mit Hilfe von 100 ml Aether in einen 500-ml-Scheidetrichter $S_1$ übergespült und dann mit 200 ml halbgesättigter Kochsalzlösung versetzt und gut geschüttelt. Zwei weitere 500-ml-Scheidetrichter ($S_2$ und $S_3$) wurden mit je 200 ml Aether beschickt. Hierauf wurden der Reihe nach die wässrige Phase aus $S_1$ sowie zwei Portionen zu je 100 ml halbgesättigter Kochsalzlösung unter gutem Durchmischen durch die drei Scheidetrichter $S_1$ bis $S_3$ geschickt. Die organischen Phasen wurden vereint und über 50 g Natriumsulfat getrocknet. Hierauf wurde abgenutscht, das Trocknungsmittel auf der Nutsche mit 50 ml Aether nachgewaschen und die Filtrate im Rotationsverdampfer am Wasserstrahlvakuum bei 30°C bis zur Gewichtskonstanz eingeengt. Man erhielt so 49,1 g (99,0%) 4-Hydroxy-4-(5-hydroxy-3-methyl-3-penten-1-ynyl)--3,5,5-trimethyl-2-cyclohexen-1-on in Form eines hellbräunlichen Oeles.

**Beispiel 2**

In einem 1-Liter-Vierhalskolben, versehen mit Rührer, Thermometer, 250-ml-Tropftrichter mit Durckausgleich und Begasungsaufsatz, wurde eine Lösung von 24,8 g (94,6 mMol) 4-Hydroxy-4-(5-hydroxy-3-methyl-3-penten-1-ynyl)-3,5,5-tri-methyl-2-cyclohexen-1-on (hergestellt gemäss Beispiel 1) in 450 ml n-Propanol/Wasser (1:1 v/v) vorgelegt. Unter Rühren und Argonbegasung wurden zunächst 13 g (47,2 mMol) Nitrilotriessigsäuretrinatriumsalz und dann 33 g (0,50 g-Atome) Zinkpulver zugesetzt. Dann wurden 300 ml (0,3 Mol) 1N-Natronlauge bei Raumtemperatur in ca. 20 Minuten zugetropft und das Gemisch anschliessend noch 4 Stunden weitergerührt. Danach wurde die Suspension unter Argon abgenutscht und der Rückstand dreimal mit je 100 ml Methylenchlorid und zweimal mit je 100 ml entionisiertem Wasser gewaschen. Das Filtrat wurde mit Hilfe von 100 ml Methylenchlorid in einen 2-Liter-Scheidetrichter $S_1$ gegeben. Zwei weitere 1-Liter-Scheidetrichter

($S_2$ und $S_3$) wurden mit je 200 ml gesättigter Kochsalzlösung beschickt. Hierauf wurden der Reihe nach und unter jeweils gutem Durchmischen zunächst die organische Phase aus $S_1$ sowie zwei Portionen zu je 200 ml Methylenchlorid durch die drei Scheidetrichter $S_1$-$S_3$ geschickt. Die organischen Phasen wurden vereint und über 100 g Natriumsulfat getrocknet. Hierauf wurde abgenutscht, der Rückstand zweimal mit je 100 ml Methylenchlorid gewaschen und die Filtrate im Rotationsverdampfer am Wasserstrahlvakuum bei einer Badtemperatur von 40°C bis zur Gewichstkonstanz getrocknet. Man erhielt 21,1 g Rohprodukt, welches identisch ist mit dem gemäss Beispiel 1 hergestellten Produkt.

**Beispiel 3**

In einem 500-ml-Mehrhalskolben, versehen mit Rührer Thermometer Tropftrichter und Begasunsaufsatz, würden unter Argon 10 g (40 mMol) 4-Hydroxy-4-(5-hydroxy-3-methyl-3-penten-1-ynyl)-3,5,5-trimethyl-2-cyclohexen-1-on (hergestellt gemäss Beispiel 1) in 200 ml n-Propanol/Wasser (1:1, v/v) gelöst und mit 20,9 g (0,32 g-Atome) Zinkpulver versetzt. Hierauf tropfte man unter Rühren 120 ml 1N NaOH-Lösung (0,12 Mol) in 5 Minuten bei Raumtemperatur zu und ruhrte noch 1 Stunde nach. Das Gemisch wurde dann in zu Beispiel 1 analoger Weise aufgearbeitet und man erhielt 9,2 g Rohprodukt. Die Reinigung an Kieselgel analog Beispiel 1 ergab 6,9 g (74,3%) 4-(5-Hydroxy-3-methyl-1,2-pentadienyl)-3,5,5-trimethyl-2-cyclohexen-1-on in Form eines Oeles. Die Struktur dieser Verbindung wurde durch Mikroanalyse, NMR-, IR- und MS-Spektren eindeutig charakterisiert.

**Beispiel 4**

A) In einem 1,5-Liter-Sulfierkolben, versehen mit Rührer, Thermometer, 500-ml-Tropftrichter mit Druckausgleich und einer Vorrichtung zur Inertbegasung, wurden 46,6 g (0,2 Mol) 4-(5-Hydroxy-3-methyl-1,3-pentadienyl)-3,5,5-trimethyl-2-cyclohexen-1-on (hergestellt gemäss Beispiel 1 oder 2) in 200 ml Methylenchlorid gelöst, vorgelegt und bei -5°C bis 0°C in ca. 20 Minuten unter gutem Rühren und Begasen mit Argon tropfenweise mit insgesamt 124 ml 37%iger Salzsäure versetzt. Hierauf wurde das Gemisch noch 15 Minuten bei 0°C bis zum vollständigen Umsatz nachgerührt. Anschliessend wurde das dunkle Reaktionsgemisch mit Hilfe von 200 ml Methylenchlorid in einen 1-Liter-Scheidetrichter $S_1$ übergeführt. Zwei weitere 1-Liter-Scheidetrichter ($S_2$ und $S_3$) wurden mit je 250 ml gesättigter Natriumbicarbonatlösung beschickt. Hierauf wurden die untere organische Phase aus $S_1$, sowie zwei Portionen zu je 250 ml Methylenchlorid unter gutem Durchmischen durch die drei Scheidetrichter $S_1$ bis $S_3$ geschickt. Die organischen Phasen wurden hierauf vereinigt, über 50 g Natriumsulfat getrocknet, abgenutscht und das Trocknungsmittel auf der Nutsche mit 50 ml Methylenchlorid nachgewaschen. Das Filtrat wurde dann im Rotationsverdampfer am Wasserstrahlvakuum bei 30°C Badtemperatur auf ein Volumen von ca. 100 ml konzentriert.

B) In einem 2,5-Liter-Sulfierkolben, versehen mit Rührer, Thermometer, 250-ml-Tropftrichter mit Druckausgleich und einer Vorrichtung zur Inertbegasung, wurde eine Lösung von 52,4 g (0,2 Mol) Triphenylphosphin in 100 ml Methylenchlorid vorgelegt. Unter Rühren und Inertbegasung wurde dann inert ca. 10 Minuten die gemäss A) erhaltene Methylenchloridlösung zugetropft. Die erhaltene dunkle Lösung wurde anschliessend noch 21 Stunden bei Raumtemperatur gerührt und dann im Rotationsverdampfer am Wasserstrahlvakuum bei 30°C Badtemperatur auf ein Volumen von ca. 200 ml konzentriert. Anschliessend wurden bei Raumtemperatur unter Rühren und Argonbegasung insgesamt 850 ml Essigester in ca. 30 Minuten zugetropft, wobei bald Auskristallisation begann. Die entstehende Suspension wurde dann 24 Stunden bei Raumtemperatur und 2 Stunden in Eis gerührt, abgenutscht, das Produkt auf der Nutsche mit zwei Portionen zu je 100 ml Essigester gründlich ausgewaschen und dann im Trockenschrank am Wasserstrahlvakuum bei 40°C bis zur Gewichtskonstanz getrocknet. Die Kristalle wurden anschliessend aus Methylenchlorid/Essigester umkristallisiert und man erhielt 63,3 g (61,7%) [3-Methyl-5-(2,6,6-trimethyl-4-oxo-2-cyclohexen-1-yl)-2,4-pentadienyl]triphenylphosphoniumchlorid. Smp. 164°-166°C.

**Beispiel 5**

A) In einem 50-ml-Mehrhalsrundkolben, versehen mit Rührer, Thermometer, 10-ml-Tropftrichter mit Druckausgleich und einer Vorrichtung zur Inertbegasungwurden 2,08 g (8,9 mMol) 4-(5-Hydroxy-3-methyl-1,3-pentadienyl)-3,5,5-trimethyl-2-cyclohexen-1-on in 10 ml Methylenchlorid gelöst, vorgelegt und bei -15°C während 10 Minuten tropfenweise mit insgesamt 4,8 ml 63%iger Bromwasserstoffsäure versetzt. Danach wurde noch während ca. 30 Minuten bei -15°C nachgerührt. Anschliessend wurde das dunkle Reaktionsgemisch mit Hilfe von 25 ml Methylenchlorid in einen 50-ml-Scheidetrichter $S_1$ transferiert. Zwei weitere 50-ml-Scheidetrichter ($S_2$ und $S_3$) wurden mit je 25 ml gesättigter Natriumbicarbonatlösung beschickt. Nun wurden die untere organische Phase aus $S_1$ sowie zwei Portionen zu je 25-ml Methylenchlorid unter gutem

Durchmischen durch die drei Scheidetrichter $S_1$ bis $S_3$ geschickt. Die organischen Phasen wurden vereint, über 25 g Natriumsulfat getrocknet, dann abgenutscht, das Trocknungsmittel auf der Nutsche mit 25 ml Methylenchlorid nachgewaschen und das Filtrat im Rotationsverdampfer am Wasserstrahlvakuum bei 30°C Badtemperatur auf ein Volumen von ca. 10 ml konzentriert.

B) In einem 50-ml-Rundkolben, versehen mit Rührer, Thermometer, 10-ml-Tropftrichter mit Druckausgleich und einer Vorrichtung zur Inertbegasung, wurden 2,89 g (11 mMol) Triphenylphosphin in 10 ml Methylenchlorid vorgelegt. Unter Rühren und Begasen mit Argon wurde dann die Methylenchloridlösung gemäss Abschnitt A) in ca. 10 Minuten zugetropft. Die dunkle Lösung wurde anschliessend während ca. 2 Stunden bei Raumtemperatur nachgerührt und dann im Rotationsverdampfer am Wasserstrahlvakuum bei 30°C Badtemperatur auf ein Volumen von ca. 20 ml konzentriert. Anschliessend wurden bei Raumtemperatur unter Rühren und Argonbegasung insgesamt 100 ml Essigester in ca. 1 Stunde zugetropft. Hierauf wurde die Suspension angeimpft, 24 Stunden bei Raumtemperatur Rückstand auf der Nutsche wurde mit zwei Portionen zu je 25 ml Essigester gründlich ausgewaschen und dann im Trockenschrank am Wasserstrahlvakuum bei 40°C bis zur Gewichtskonstanz getrocknet. Man erhielt 4,2 g (84,3%) [3-Methyl-5-(2,6,6-trimethyl-4-oxo-2-cyclohexen-1-yl)-2,4-pentadienyl]triphenylphosphonium bromid. Smp. 168°-170°C.

**Beispiel 6**

A) In einem 1,5-Liter-Sulfierkolben, versehen mit Rührer, Thermometer, 50-ml-Tropftrichter mit Druckausgleich und einer Vorrichtung zur Inertbegasung, wurden 103 g (0,20 Mol) [3-Methyl-5-(2,6,6-trimethyl-4-oxo-2-cyclohexen-1-yl)-2,4-pentadienyl]triphenylphosphonium-chlorid und 13,1 g (79,8 mMol) 2,7-Dimethyl-octatrien-(2,4,6)-dial-(1,8), in 800 ml Methylenchlorid gelöst, vorgelegt. Unter Rühren, Begasung mit Argon und Kühlen bei -20°C wurden nun im Verlauf von zwei Stunden insgesamt 46 ml 10%ige Natriummethylatlösung zugetropft. Anschliessend wurde noch zwei Stunden bei 0°C nachgerührt. Die Reaktion wurde durch Zusatz von 12 ml Eisessig gequencht. Drei 2-Liter-Scheidetrichter $S_1$ bis $S_3$ wurden mit je 500 ml gesättigter Kochsalzlösung beschickt. Dann wurden unter kräftigem Durchmischen zunächst die Reaktionslösung und dann zwei Portionen zu je 500 ml Methylenchlorid durch die drei Scheidetrichter $S_1$ bis $S_3$ geschickt. Die organischen Extrakte wurden vereinigt, über 100 g Natriumsulfat getrocknet, abgenutscht und das Trocknungsmittel auf der Nutsche mit 2mal 200 ml Methylenchlorid nachgewaschen.

B) Das gemäss A) erhaltene Filtrat wurde in einen 1,5-Liter-Sulfierkolben, versehen mit Rührer, Thermometer, 250-ml-Tropftrichter mit Druckausgleich und Vorrichtung für Inertbegasung, sowie einem Destillationsaufsatz, gegeben. Unter Rühren und Heizen (120°C) und unter Argon wurde die Lösung bei Normaldruck auf ein Volumen von ca. 500 ml konzentriert. Dann wurden unter fortgesetzter Destillation bei konstantem Volumen von ca. 500 ml etwa 1,8 l Methanol so lange zugetropft, bis der Siedepunkt von 63°C erreicht wurde. Während dieses Vorganges kristallisierte ein Produkt aus. Anschliessend wurde der Destillationsaufsatz durch einen Rückflusskühler ersetzt und die Suspension während 3 Tagen am Rückfluss gerührt. Dann wurde das Ganze auf -20°C gekühlt und abgenutscht. Die Kristalle wurde auf der Nutsche mit drei Portionen zu je 100 ml Methanol (von -20°C) unter Argonbegasung gründlich ausgewaschen, abgepresst und im Trockenschrank unter Wasserstrahlvakuum bei 30°C bis zur Gewichtskonstanz getrocknet. Man erhielt 32 g (71%) 1,18-bis(4-oxo-2,6,6-trimethyl-2-cyclohexen-1-yl)-3,7,12,16-tetramethyl-1,3,5,7,9,11,13,15,17-octadecanonaen. Schmelzpunkt 185°-187°C.

In zum Vorhergehenden analoger Weise kann diese Verbindung auch ausgehend von [3-Methyl-5-(2,6,6-trimethyl-4-oxo-2 -cyclo-hexen-1-yl)-2,4-pentadienyl]triphenylphosphonium-bromid hergestellt werden.

**Beispiel 7**

A) In einem 500-ml-Mehrhalskolben, versehen mit Magnetrührkern, 50-ml-Tropftrichter und Thermometer, wurden 11,3 g (20 mMol) 1,18-bis(4-oxo-2,6,6-trimethyl-2 -cyclo-hexen-1-yl)-3,7,12,16-tetramethyl-1,3,5,7,9,11,13,15,17-octadecanonaen in 200 ml Pyridin bei 70°C unter Argon gelöst, auf Raumtemperatur gekühlt, unter Luftzutritt 20 Minuten bei 0°C gerührt, und dann tropfenweise während 10 Minuten mit 50 ml einer 1N-äthanolischen Kaliumhydroxidlösung versetzt. Dann wurde noch während 30 Minuten in Eis und während 1,5 Stunden bei Raumtemperatur und freiem Luftzutritt gerührt. Die Reaktionslösung wurde dann mit Hilfe von 200 ml Methylenchlorid in einen 1-Liter-Scheidetrichter $S_1$ transferiert, welcher 400 ml einer 5%igen Kochsalzlösung enthielt. Zwei weitere 1-Liter-Scheidetrichter ($S_2$ und $S_3$) wurden mit je 500 ml 3N Salzsäure beschickt. Nun wurden die unter, organische Phase aus $S_1$ sowie drei Portionen zu je 200 ml Methylenchlorid unter gutem Durchmischen durch die drei Scheidetrichter ($S_1$ bis $S_3$) geschickt. Die organischen Extrakte wurden mit 400 ml entionisiertem Wasser und 400 ml 2%iger Natriumbicarbonatlösung gewaschen, über 100 ml Natriumsulfat getrocknet und abgenutscht. Das Trocknungsmittel wurde auf der Nutsche mit zwei Portionen zu je 200 ml Methylenchlorid nachgewaschen und das Filtrat am Rotationsverdampfer am Wasserstrahlvakuum bei 30°C auf ein Volumen von ca. 100 ml konzentriert.

B) Die gemäss A) erhaltene Methylenchloridlösung (100 ml) wurde in einen 350-ml-Sulfierkolben, versehen

mit Rührer, Thermometer, 100-ml-Tropftrichter mit Druckausgleich und Vorrichtung für Inertbegasung sowie einem Destillationsaufsatz, gegeben. Unter Rühren und Destillation wurde bei Normaldruck bis auf ein Restvolumen von ca. 100 ml konzentriert. Unter Beibehaltung dieses Volumens wurde anschliessend soviel entionisiertes Wasser zugetropft, bis der Siedepunkt 93°C (Badtemperatur = 130°C) erreicht war. Die entstandene klebrige Masse wurde dann 48 Stunden am Rückfluss gerührt, auf Raumtemperatur gekühlt und dann mit Hilfe von ca. 200 ml Methylenchlorid gelöst. Nach dem Trennen der Phasen in einem 500-ml-Scheidetrichter wurde die organische Phase über 25 g Natriumsulfat getrocknet und abgenutscht. Das Trocknungsmittel wurde auf der Nutsche mit zwei Portionen zu je 100 ml Methylenchlorid nachgewaschen und das Filtrat am Rotationsverdampfer am Wasserstrahlvakuum bei 30°C auf ein Volumen von ca. 50 ml konzentriert. Diese Lösung wurde mit 150 ml Essigester versetzt, worauf das Gemisch im Rotationsverdampfer am Wasserstrahlvakuum bei 40°C Badtemperatur auf ein Volumen von ca. 50 ml konzentriert wurde. Hierbei begann Produkt auszukristallisieren. Die Suspension wurde anschliessend noch zwei Stunden bei 0°C gerührt und dann genutscht. Die Kristalle auf der Nutsche wurden mit zwei Portionen zu je 50 ml Essigester gründlich ausgewaschen und dann im Trockenschrank am Wasserstrahlvakuum bei 30°C bis zur Gewichtskonstanz getrocknet. Man erhielt 6,3 g (56%) Rhodoxanthin mit einem Schmelzpunkt von 208°-210°C.

**Patentansprüche**

füur die Vertragsstaaten BE CH DE FR GB IT LI NL

1. Verfahren zur Herstellung von Cyclohexenderivaten, dadurch gekennzeichnet, dass man die Verbindung der Formel

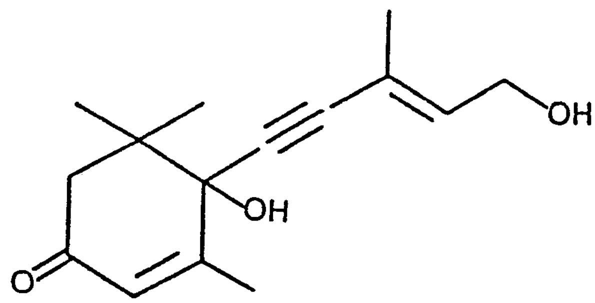

I

in einem stark basischen, homogenen, wässrig-organischen Lösungsmittelgemisch mit Zink in die Verbindung der Formel

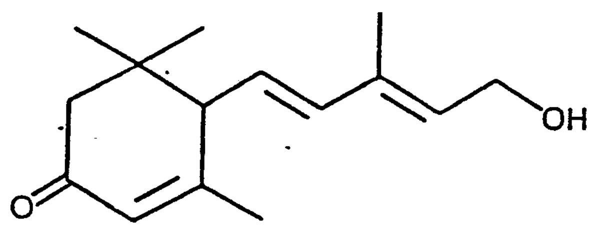

II

überführt, dass man, gewünschtenfalls, die Verbindung der Formel II, nach Ueberführung in das Phosphoniumsalz der Formel

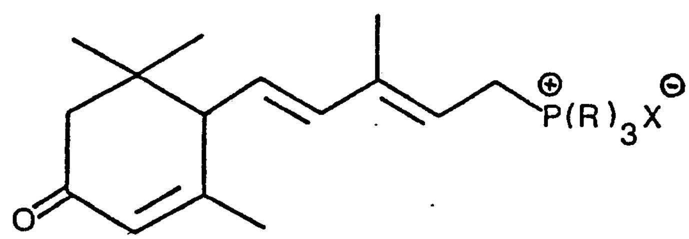

III

worin R Phenyl und X Chlor, Brom oder Jod bedeuten, mit dem Dialdehyd der Formel

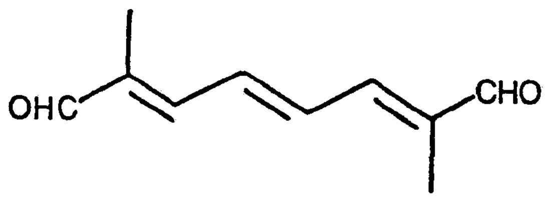

IV

zur Verbindung der Formel

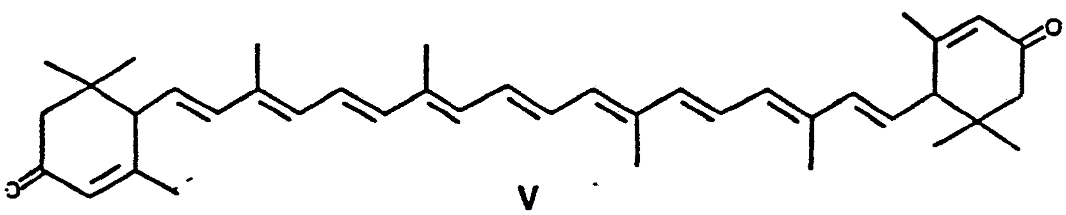

V

umsetzt, dass man, gewünschtenfalls, die Verbindung der Formel V zu Rhodoxanthin der Formel

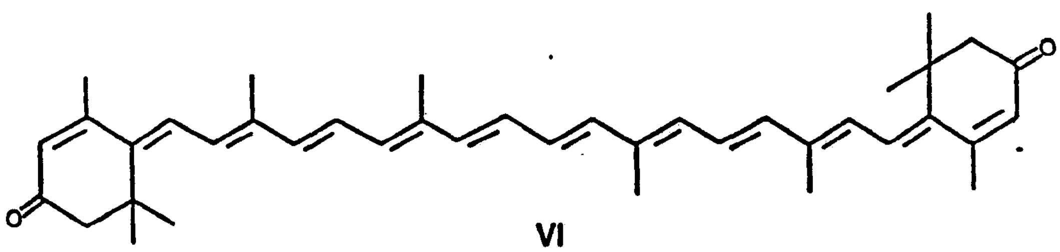

VI

dehydriert und dass man, gewünschtenfalls, dieses Rhodoxanthin zu Zeaxanthin der Formel

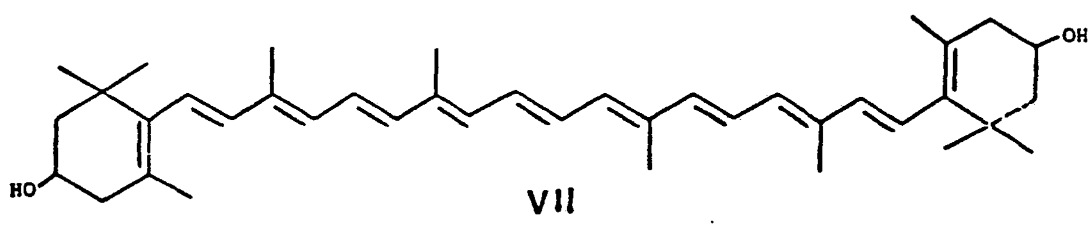

VII

reduziert.

2. Verfahren nach Anspruch 1, dadurch gekennzeichnet, dass man die Umsetzung der Verbindung der Formel I zu derjenigen der Formel II bei einem pH-Wert von etwa 13-14, vorzugsweise bei pH 14 durchführt.

3. Verfahren nach Anspruch 1 oder 2, dadurch gekennzeichnet, dass man als Lössungsmittel in dem wässrigorganischen Lösungsmittelgemisch einen niederen Alkohol mit 1 bis 4 Kohlenstoffatomen, Dioxan, Tetrahydrofuran, Aceton, Dimethylsulfoxid oder Dimethylformamid verwendet.

4. Verfahren nach Anspruch 3, dadurch gekennzeichnet, dass man als Lösungsmittel einen niederen Alkohol mit 1 bis 4 Kohlenstoffatomen, vorzugsweise Methanol, Aethanol oder n-Propanol verwendet.

5. Verfahren nach einem der Ansprüchr 1 bis 4, dadurch gekennzeichnet, dass man die Umsetzung der Verbindung der Formel I zu derjenigen der Formel II in Gegenwart eines Komplexbildners durchführt.

6. Verfahren nach Anspruch 5, dadurch gekennzeichnet, dass man als Komplexbildner ein Alkalicyanid, Aethylendiamintetraessigsäure, Aethylendiamin, Nitrilotriessigsäure, oder o-Phenanthrolin verwendet.

7. Verfahren nach Anspruch 5 oder 6, dadurch gekennzeichnet, dass man als Komplexbildner Natriumcyanid, Kaliumcyanid, Nitrilotriessigsäure, Aethylendiamintetraessigsäure oder Aethylendiamin verwendet.

8. Verfahren nach einem der Ansprüche 1 bis 7, dadurch gekennzeichnet, dass man die Ueberführung der Verbindung der Formel I in diejenige der Formel II unter Inertgasatmosphäre durchführt.

9. Verbindungen der Formel

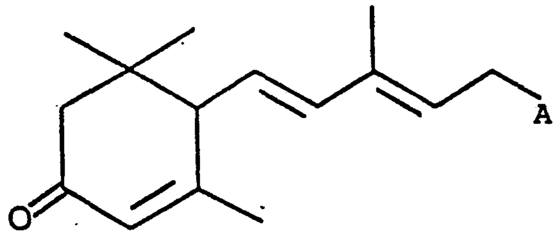

II A

worin A die Hydroxygruppe oder die Gruppe $P^{\oplus}(R)_3X^{\ominus}$ darstellt und R Phenyl und X Chlor, Brom oder jod bedeuten.

**Patentansprüch**

für den Vertragsstaat AT

1. Verfahren zur Herstellung von Cyclohexenderivaten, dadurch gekennzeichnet, dass man die Verbindung der Formel

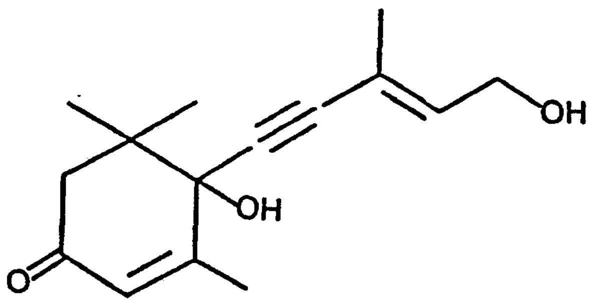

I

in einem stark basischen, homogenen, wässrig-organischen Lösungsmittelgemisch mit Zink in die Verbindung der Formel

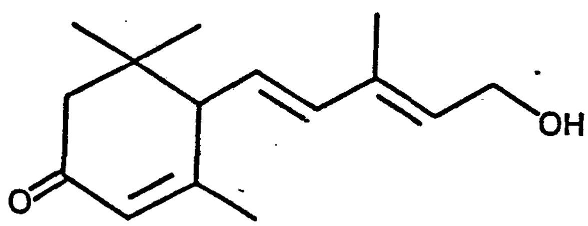

II

überführt, dass man, gewünschtenfalls, die Verbindung der Formel II, nach Ueberführung in das Phosphoniumsalz der Formel

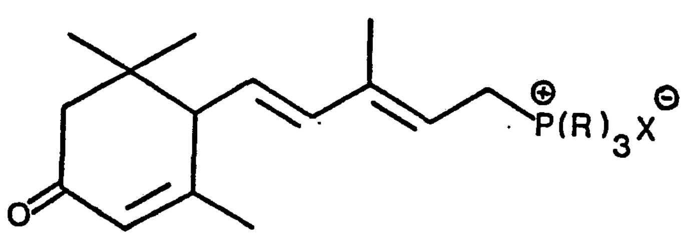

III

worin R Phenyl und X Chlor, Brom oder Jod bedeuten, mit dem Dialdehyd der Formel

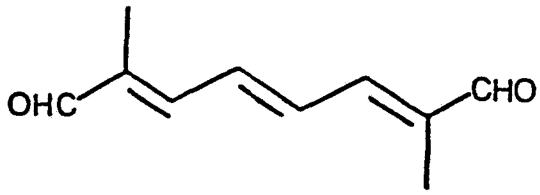

IV

zur Verbindung der Formel

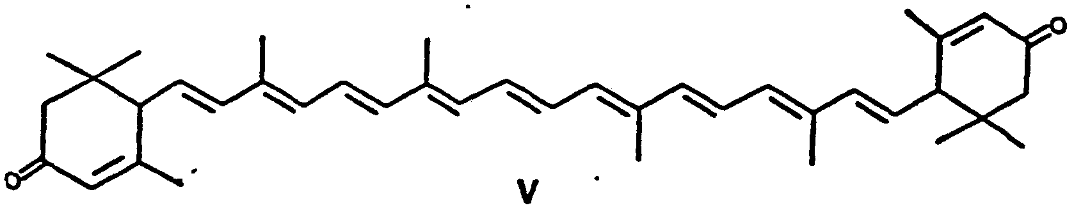

V

umsetzt, dass man, gewünschtenfalls, die Verbindung der Formel V zu Rhodoxanthin der Formel

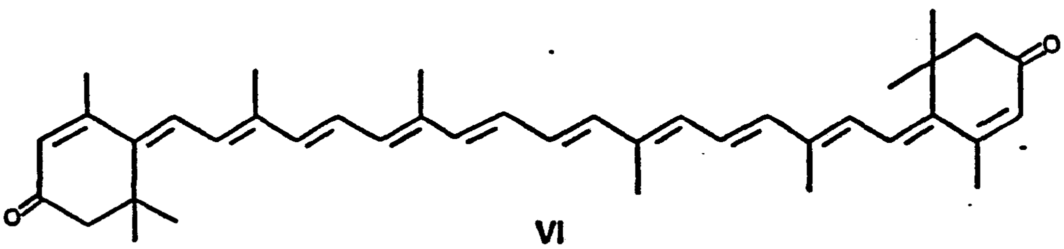

VI

dehydriert und dass man, gewünschtenfalls, dieses Rhodoxanthin zu Zeaxanthin der Formel

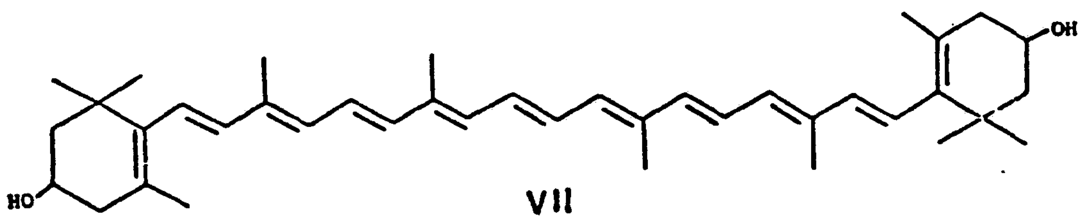

VII

reduziert.

2. Verfahren nach Anspruch 1, dadurch gekennzeichnet, dass man die Umsetzung der Verbindung der Formel I zu derjenigen der Formel II bei einem pH-Wert von etwa 13-14, vorzugsweise bei pH 14 durchführt.

3. Verfahren nach Anspruch 1 oder 2, dadurch gekennzeichnet, dass man als Lösungsmittel in dem wässrigorganischen Lösungsmittelgemisch einen niederen Alkohol mit 1 bis 4 Kohlenstoffatomen, Dioxan, Tetrahydrofuran, Aceton, Dimethylsulfoxid oder Dimethylformamid verwendet.

4. Verfahren nach Anspruch 3, dadurch gekennzeichnet, dass man als Lösungsmittel einen niederen Alkohol mit 1 bis 4 Kohlenstoffatomen, vorzugsweise Methanol, Aethanol oder n-Propanol verwendet.

5. Verfahren nach einem der Ansprüche 1 bis 4, dadurch gekennzeichnet, dass man die Umsetzung der Verbindung der Formel I zu derjenigen der Formel II in Gegenwart eines Komplexbildners durchführt.

6. Verfahren nach Anspruch 5, dadurch gekennzeichnet, dass man als Komplexbildner ein Alkalicyanid, Aethylendiamintetraessigsäure, Aethylendiamin, Nitrilotriessigsäure, Sulfide oder o-Phenanthrolin verwendet.

7. Verfahren nach Anspruch 5 oder 6, dadurch gekennzeichnet, dass man als Komplexbildner Natriumcyanid, Kaliumcyanid, Nitrilotriessigsäure, Aethylendiamintetraessigsäure oder Aethylendiamin verwendet.

8. Verfahren nach einem der Ansprüche 1 bis 7, dadurch gekennzeichnet, dass man die Ueberführung der Verbindung der Formel I in diejenige der Formel II unter Inertgasatmosphäre durchführt.

**Claims**

for the Contracting States BE CH DE FR GB IT LI NL

1. A process for the manufacture of cyclohexene derivatives, characterized by converting the compound of the formula

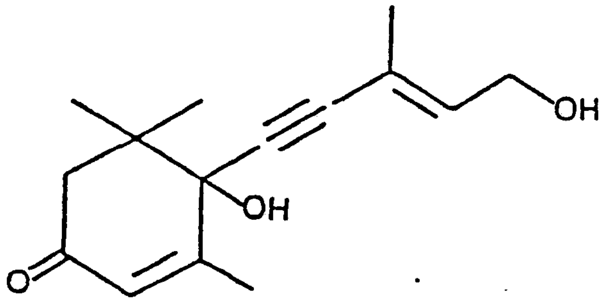

I

with zinc in a strongly basic, homogeneous, aqueous-organic solvent mixture into the compound of the formula

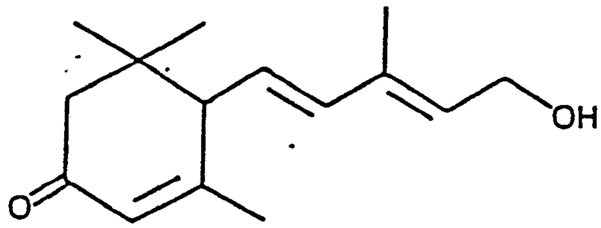

II,

if desired, reacting the compound of formula II, after conversion into the phosphonium salt of the formula

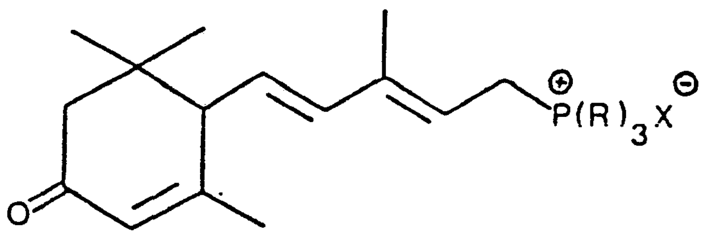

III

wherein R signifies phenyl and X signifies chlorine, bromine or iodine,
with the dialdehyde of the formula

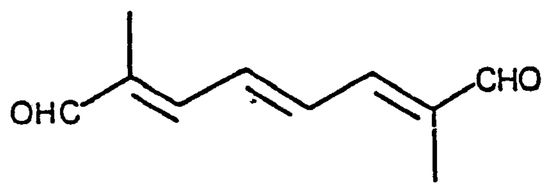

IV

to give the compound of the formula

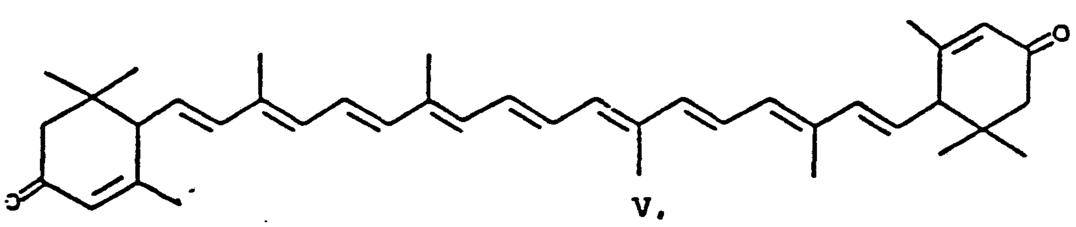

V,

if desired, dehydrogenating the compound of formula V to rhodoxanthin of the formula

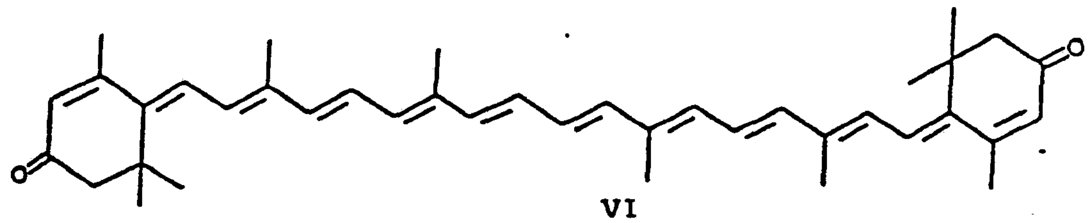

VI

and, if desired, reducing this rhodoxanthin to zeaxanthin of the formula

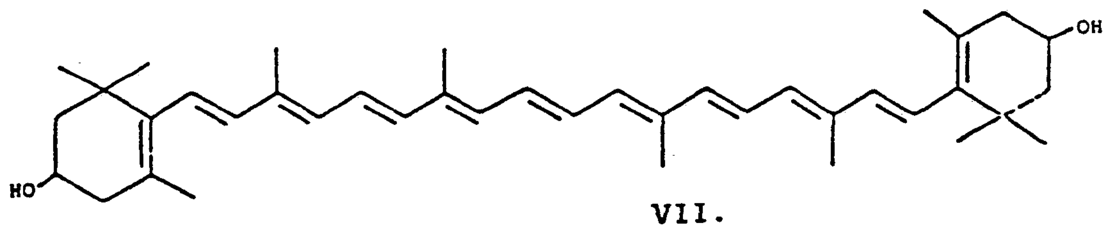

VII.

2. A process according to claim 1, characterized in that the conversion of the compound of formula I into that of formula II is carried out at a pH of about 13-14, preferably at pH 14.

3. A process according to claim 1 or 2, characterized in that a lower alcohol with 1 to 4 carbon atoms, dioxan, tetrahydrofuran, acetone, dimethyl sulphoxide or dimethylformamide is used as the solvent in the aqueous-organic solvent mixture.

4. A process according to claim 3, characterized in that a lower alcohol with 1 to 4 carbon atoms, preferably methanol, ethanol or n-propanol, is used as the solvent.

5. A process according to any one of claims 1l to 4, characterized in that the conversion of the compound of formula I into that of formula II is carried out in the presence of a complex former.

6. A process according to claim 5, characterized in that an alkali cyanide, ethylenediaminetetraacetic acid, ethylenediamine, nitrilotriacetic acid or o-phenanthroline is used as the complex former.

7. A process according to claim 5 or 6, characterized in that sodium cyanide, potassium cyanide, nitrilotriacetic acid, ethylenediaminetetraacetic acid or ethylenediamine is used as the complex former.

8. A process according to any one of claims 1 to 7. characterized in that the conversion of the compound of formula I into that of formula II is carried out under an inert gas atmosphere.

9. Compounds of the formula

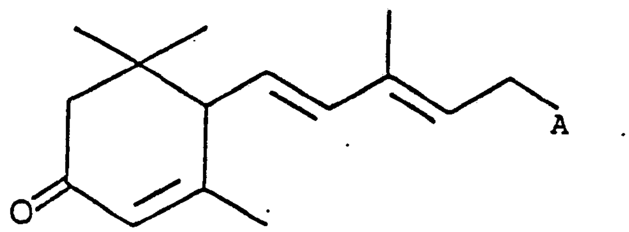

IIA

wherein A represents the hydroxy group or the group $p^{\oplus}(R)_3X^{\ominus}$ and R signifies phenyl and X signifies chlorine, bromine or iodine.

**Claims**

for the Contracting State AT

1. A process for the manufacture of cyclohexene derivatives, characterized by converting the compound of the formula

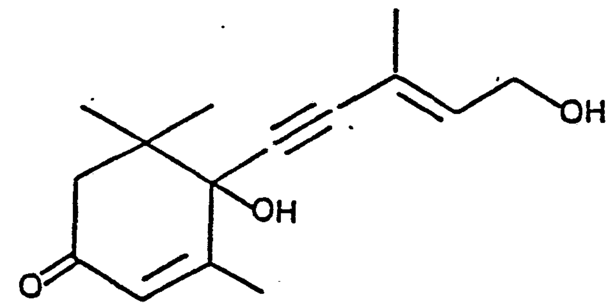

I

with zinc in a strongly basic, homogeneous, aqueous-organic solvent mixture into the compound of the formula

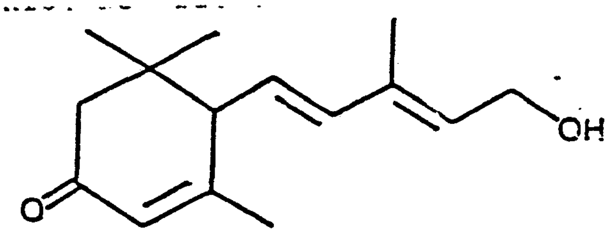

II,

if desired, reacting the compound of formula II, after conversion into the phosphonium salt of the formula

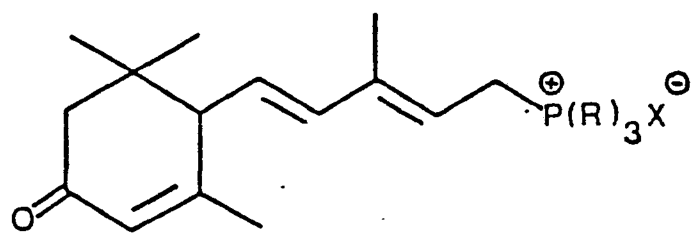

III

wherein R signifies phenyl and X signifies chlorine, bromine or iodine. with the dialdehyde of the formula

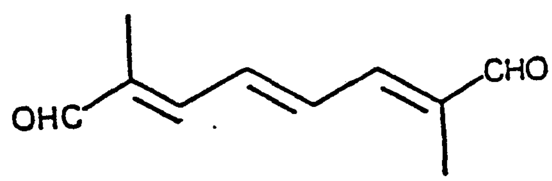

IV

to give the compound of the formula

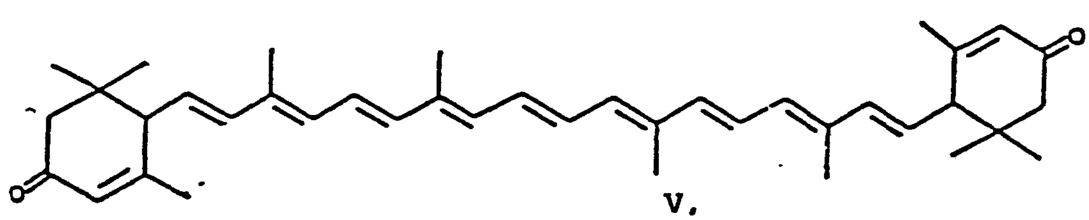

V,

if desired, dehydrogenating the compound of formula V to rhodoxanthin of the formula

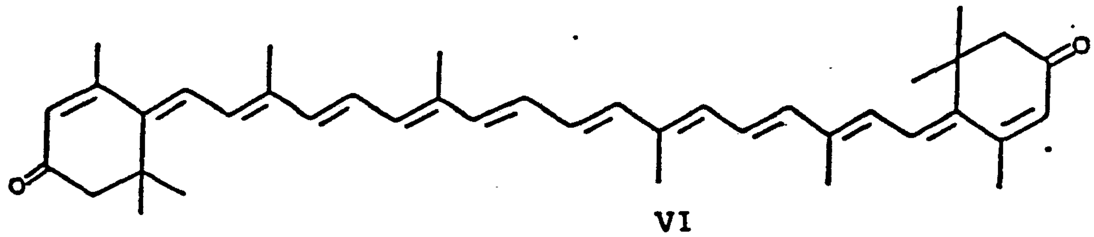

VI

and, if desired, reducing this rhodoxanthin to zeaxanthin of the formula

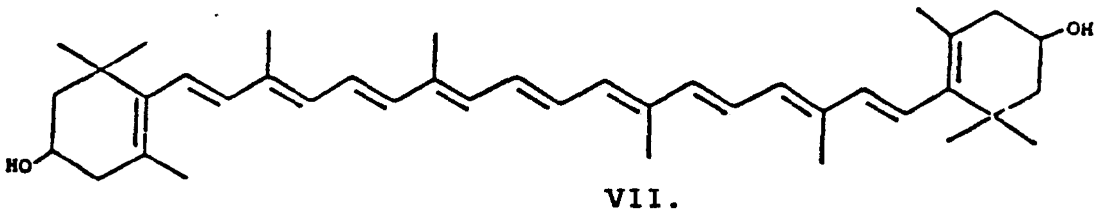

VII.

2. A process according to claim 1, characterized in that the conversion of the compound of formula I into that of formula II is carried out at a pH of about 13-14, preferably at pH 14.

3. A process according to claim 1 or 2, characterized in that a lower alcohol with 1 to 4 carbon atoms, dioxan, tetrahydrofuran, acetone, dimethyl sulphoxide or dimethylformamide is used as the solvent in the aqueous-organic solvent mixture.

4. A process according to claim 3, characterized in that a lower alcohol with I to 4 carbon atoms, preferably methanol, ethanol or n-propanol, is used as the solvent.

5. A process according to any one of claims 1 to 4, characterized in that the conversion of the compound of formula I into that of formula II is carried out in the presence of a complex former.

6. A process according to claim 5, characterized in that an alkali cyanide, ethylenediaminetetraacetic acid, ethylenediamine. nitrilotriacetic acid, a sulphide or o-phenanthroline is used as the complex former.

7. A process according to claim 5 or 6, characterized in that sodium cyanide, potassium cyanide, nitrilotriacetic acid, ethylenediaminetetraacetic acid or ethylenediamine is used as the complex former.

8. A process according to any one of claims 1 to 7. characterized in that the conversion of the compound of formula I into that of formula II is carried out under an inert gas atmosphere.

**Revendications**

pour les Etats contractants BE CH DE FR GB IT LI NL

1. Procedé de préparation de dérivés du cyclohéxène caractérisé en ce que l'on convertit le composé de formule

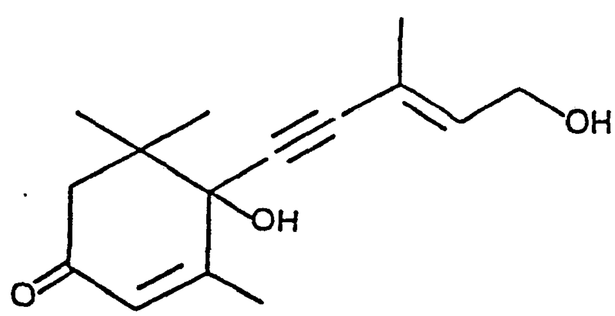

I

dans un mélange de solvants hydro-organique, homogène, fortement basique, avec du zinc, en le composé de formule

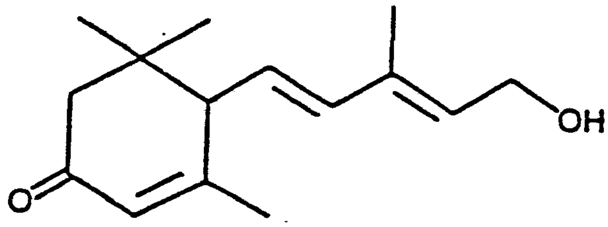

II

que l'on fait réagir, le cas échéant, le composé de formule II, après conversion en le sel de phosphonium de formule

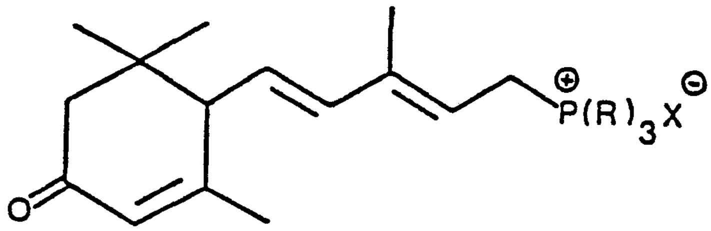

III

dans laquelle R représente le phényl et X le chlore, le brome ou l'iode, avec le dialdéhyde de formule

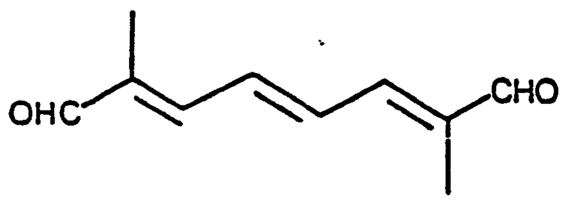

IV

en vue de l'obtention du composé de formule

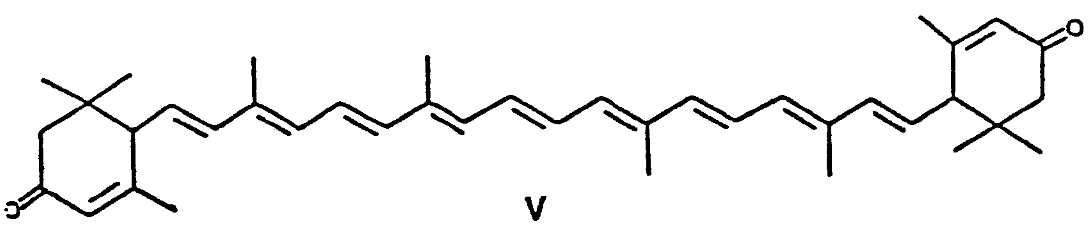

V

que l'on déshydrogéne, le cas échéant, le compose de formule V en la rhodoxanthine de formule

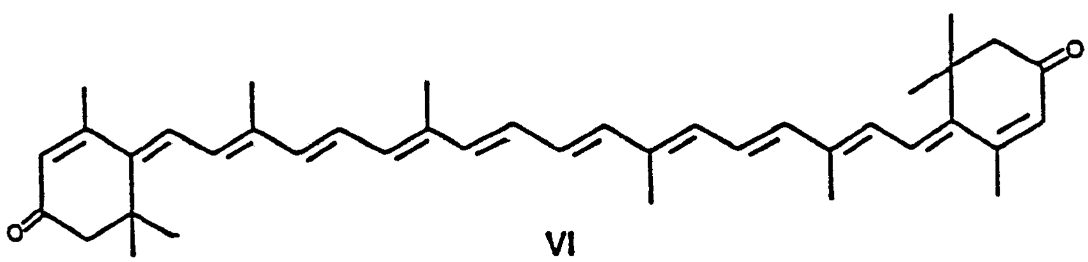

VI

et que l'on réduit, le cas échéant, cette rhodoxanthine en la zéaxanthine de formule

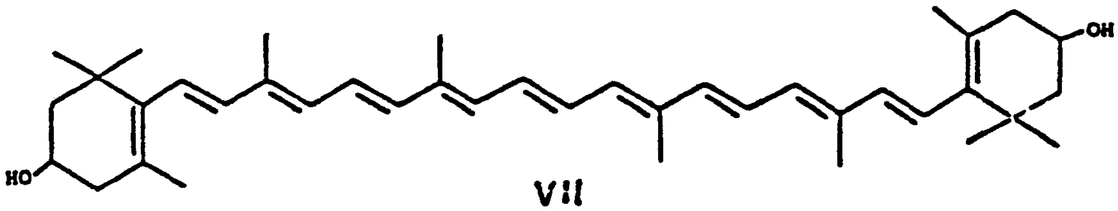

VII

2. Procédé selon la revendication 1, caractérisé en ce qu'on conduit la réaction du composé de formule I en celuide formule II à une valeur de pH d'environ 13-14, de préférence à pH 14.

3. Procédé selon la revendication 1 ou 2, caractérisé en ce qu'on utilise comme solvants dans le mélange de solvants hydro-organique, un alcool inférieur avec 1 à 4 atomes de carbone, du dioxanne, du tétrahydrofuranne, de l'acétone, du diméthylsulfoxyde ou du diméthylformamide.

4. Procédé selon la revendication 3, caractérisé en ce qu'on utilise comme solvant un alcool inférieur avec 1 à 4 atomes de carbone, de préférence le méthanol, l'éthanol ou le n-propanol.

5. Procédé selon l'une des revendications 1 à 4, caractérisé en ce qu'on effectue la transformation du composé de formule I en celui de formule II en présence d'un agent complexant.

6. Procédé selon la revendication 5, caractérisé en ce qu'on utilise comme complexant un cyanure alcalin, de l'acide éthylènediaminetétraacétique, de l'éthylènediamine, de l'acide nitrilotriacétique ou de l'o-phénanthroline.

7. Procédé selon la revendication 5 ou 6, caractérisé en ce qu'on utilise comme complexant du cyanure de sodium, du cyanure de potassium, de l'acide nitrilotriacétique, de l'acide éthylènediaminetétraacétique ou de l'éthylènediamine.

8. Procédé selon l'une des revendications 1 à 7, caractérisé en ce qu'on effectue la transformation du composé de formule I en celui de formule II sous atmosphère de gaz inerte.

9. Composés de formule

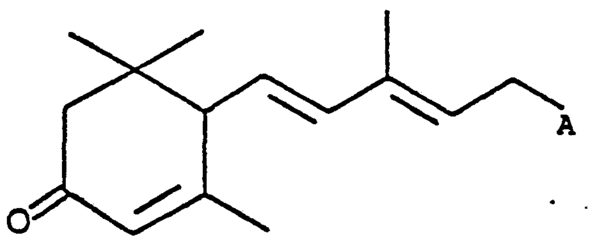

II A

dans laquelle A représente le groupe hydroxyle ou le groupe $P^{\oplus}(R)_3X^{\ominus}$ et R représente un phényle et X le chlore, le brome ou l'iode.

**Revendications**

pour l'Etat contractant AT

1. Procédé de préparation de dérivés du cyclohéxène, caractérisé en ce que l'on convertit le composé de formule

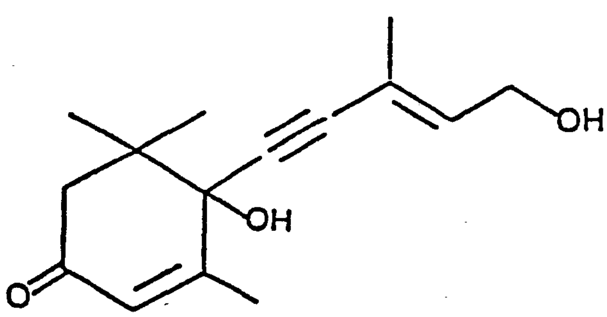

I

dans un mélange de solvants hydro-organique, homogène, fortement basique, avec du zinc, en le composé de formule

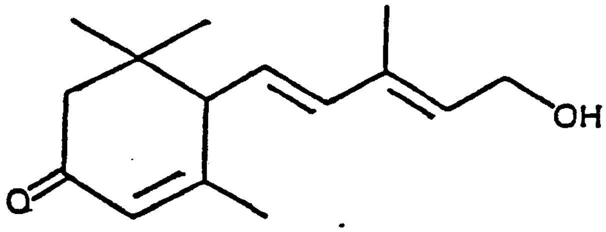

II

que l'on fait réagir, le cas échéant, le composé de formule II, après conversion en le sel de phosphonium de formule

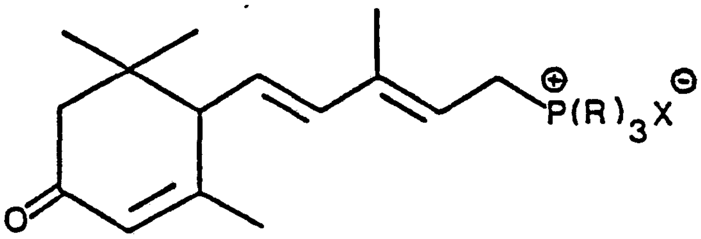

III

dans laquelle R représente le phényl et X le chlore, le brome ou l'iode, avec le dialdéhyde de formule

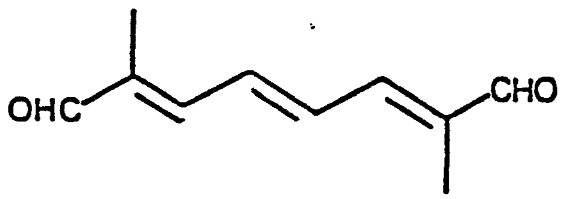

IV

en vue de l'obtention du composé de formule

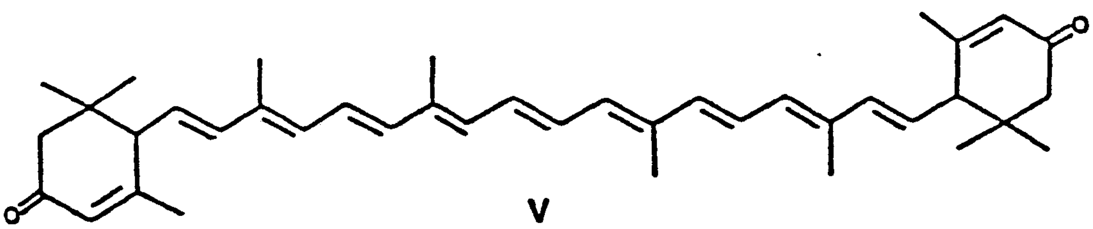

V

que l'on déshydrogène, le cas échéant, le compose de formule V en la rhodoxanthine de formule

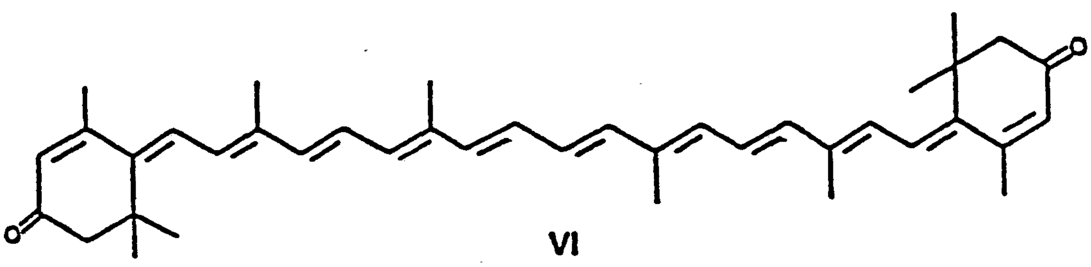

VI

et que l'on réduit, le cas échéant, cette rhodoxanthine en la zéaxanthine de formule

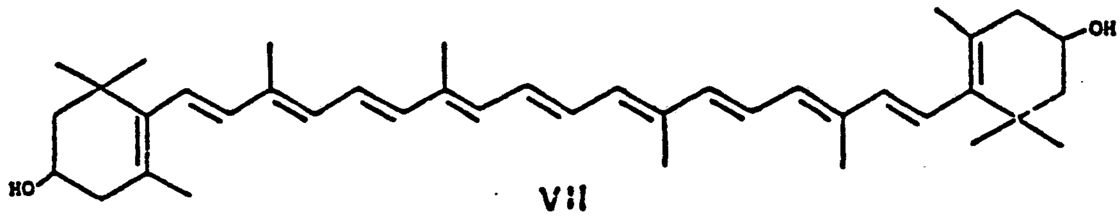

VII

2. Procédé selon la revendication 1, caractérisé en ce qu'on conduit la réaction du composé de formule I en celui de formule II à une valeur de pH d'environ 13-14, de préférence à pH 14.

3. Procédé selon la revendication 1 ou 2, caractérisé en ce qu'on utilise comme solvants dans le mélange de solvants hydro-organique, un alcool inférieur avec 1 à 4 atomes de carbone, le dioxanne, le tétrahydrofuranne, l'acétone, le diméthylsulfoxyde ou le diméthylformamide.

4. Procédé selon la revendication 3, caractérisé en ce qu'on utilise comme solvant un alcool inférieur avec 1 à 4 atomes de carbone, de préférence le méthanol, l'éthanol ou le n-propanol.

5. Procédé selon l'une des revendications 1 à 4 caractérisé en ce qu'on effectue la transformation du composé de formule I en celui de formule II en présence d'un agent complexant.

6. Procédé selon la revendication 5, caractérisé en ce qu'on utilise comme complexant un cyanure alcalin, de l'acide éthylènediaminetétraacétique, de l'éthylènediamine, de l'acide nitrilotriacétique ou de l'o-phénanthroline.

7. Procédé selon la revendication 5 ou 6, caractérisé en ce qu'on utilise comme complexant du cyanure de sodium, du cyanure de potassium, de l'acide nitrilotriacétique, de l'acide éthylènediaminetétraacétique ou de l'éthylènediamine.

8. Procédé selon l'une des revendications 1 à 7, caractérisé en ce qu'on effectue la transformation du composé de formule I en celui de formule II sous atmosphère de gaz inerte.